# EUROPEAN PATENT APPLICATION

(11) **EP 3 467 762 A1**
(43) Date of publication of application: **10.04.2019**
(21) Application number: 17802819.7
(22) Date of filing: 23.05.2017
(51) Int. Cl.: G06Q 50/22, A61B 5/00

(54) **HEALTH CONDITION PREDICTION DEVICE, HEALTH CONDITION PREDICTION METHOD, AND COMPUTER-READABLE RECORDING MEDIUM**

(30) Priority: 23.05.2016 JP 2016102719
(71) Applicant: NEC Solution Innovators, Ltd., Koto-ku, Tokyo 136-8627 (JP)
(72) Inventor: TANAKA, Hirofumi, Tokyo 136-8627 (JP); NAKAMICHI, Masashi, Tokyo 136-8627 (JP); FUKUNISHI, Hiroaki, Tokyo 108-8001 (JP)
(74) Representative: Betten & Resch
(86) International application number: PCT/JP2017/019288
(87) International publication number: WO 2017/204233

(57) **Abstract**

The health condition prediction apparatus 10 includes an estimation model learning unit 11 for learning a model indicating a relationship between lifestyle and a check value for a preset check item, using actual data regarding individuals' lifestyle and the check value as training data, a check value prediction unit 12 for acquiring actual data regarding lifestyle of a user and predicting a future check value of the user by using the acquired actual data and the model, and a display unit 13 for displaying, on a screen, the future check value predicted by the check value prediction unit 12.

## Description

### TECHNICAL FIELD

The present invention relates to a health condition prediction apparatus and a health condition prediction method for predicting a future health condition based on the current health condition of a user, and further relates to a computer-readable recording medium storing a program for realizing these.

### BACKGROUND ART

In recent years, major increases in medical costs due to lifestyle-related diseases are a major problem for corporate health insurance organizations. To take measures against lifestyle-related diseases, corporations place medical staff such as industrial physicians or public health nurses to enhance measures directed toward medical checks and health guidance for employees.

Also, in recent years, big data in the field of medical and health care has been more actively used for measures against lifestyle-related diseases. The Ministry of Health, Labour and Welfare has demanded, since the 2015 fiscal year, that all health insurance organizations create and carry out data health plans. It is envisioned that the future health condition of individual employees can be predicted, and specific improvement measures can be recommended, for example, through big data analysis.

For example, Patent Document 1 discloses a system that simulates a personal health index and health risk, based on personal check value data and lifestyle data. The system disclosed in Patent Document 1 can present a health age and healthy life expectancy in the case if a user were to stop smoking or reduce their alcohol intake, for example. Accordingly, with the system disclosed in Patent Document 1, an industrial physician, a public health nurse, or the like, can readily recommend specific improvement measures for employees.

### LIST OF PRIOR ART DOCUMENTS

### PATENT DOCUMENT

Patent Document 1: JP 2014-119817A

### DISCLOSURE OF THE INVENTION

### PROBLEMS TO BE SOLVED BY THE INVENTION

However, although employees receive heath guidance to improve their lifestyle, it is difficult to urge employees to change their behavior, which is a major problem. Moreover, although the system disclosed in Patent Document 1 presents a health age and healthy life expectancy to a user assuming an improvement in lifestyle, these indices lack reality, and the foregoing problem has not yet been solved by using this system.

An example of an object of the present invention is to solve the foregoing problem and provide a health condition prediction apparatus, a health condition prediction method, and a computer-readable recording medium capable of making a user realize that his/her health condition will change as a result of improving his/her lifestyle.

### MEANS FOR SOLVING THE PROBLEMS

To achieve the above-stated object, a health condition prediction apparatus according to an aspect of the present invention includes:
an estimation model learning unit for learning a model indicating a relationship between lifestyle and a check value for a preset check item, using actual data regarding individuals' lifestyle and the check value as training data;
a check value prediction unit for acquiring actual data regarding lifestyle of a user, and predicting a future check value of the user by using the acquired actual data and the model; and
a display unit for displaying, on a screen, the future check value predicted by the check value prediction unit.

Also, to achieve the above-stated object, a health condition prediction method according to an aspect of the present invention includes:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b).

Furthermore, to achieve the above-stated object, a computer-readable recording medium according to an aspect of the present invention stores a program including a command for causing a computer to perform:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b).

### ADVANTAGEOUS EFFECTS OF THE INVENTION

As described above, the present invention can make a user aware that his/her health condition will change as a result of improving his/her lifestyle.

### BRIEF DESCRIPTION OF THE DRAWINGS

FIG. 1 is a block diagram illustrating a schematic configuration of a health condition prediction apparatus according to an embodiment of the present invention.
FIG. 2 is a block diagram illustrating a specific configuration of the health condition prediction apparatus according to the embodiment of the present invention.
FIG. 3 is a flowchart illustrating an operation at the time of processing to predict check values performed by the health condition prediction apparatus according to the embodiment of the present invention.
FIG. 4 shows an example of actual data that is acquired from a user.
FIG. 5 shows an example of prediction results.
FIG. 6 shows an example of results of determining the risk of suffering from lifestyle-related diseases.
FIG. 7 is a flowchart illustrating operations during processing to predict post-change check values performed by the health condition prediction apparatus according to the embodiment of the present invention.
FIG. 8 shows an input example of a changed lifestyle.
FIG. 9 illustrates an example of results of predicting post-change check values.
FIG. 10 is a block diagram illustrating a configuration of the health condition prediction apparatus according to Modification 1 of the embodiment of the present invention.
FIG. 11 is a block diagram illustrating an example of a computer that realizes the health condition prediction apparatus according to the embodiment of the present invention.

### MODE FOR CARRYING OUT THE INVENTION

### Embodiment

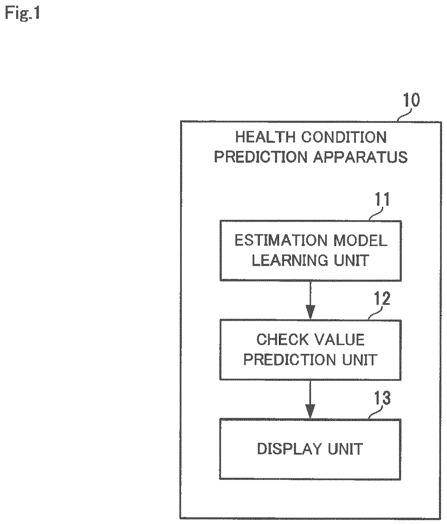

Hereinafter, a health condition prediction apparatus, a health condition prediction method, and a program according to an embodiment of the present invention will be described with reference to FIGS. 1 to 11.

### Apparatus configuration

First, a configuration of the health condition prediction apparatus according to this embodiment will be described with reference to FIG. 1. FIG. 1 is a block diagram illustrating a schematic configuration of the health condition prediction apparatus according to an embodiment of the present invention.

As shown in FIG. 1, a health condition prediction apparatus 10 according to this embodiment includes an estimation model learning unit 11, a check value prediction unit 12, and a display unit 13. The estimation model learning unit 11 learns a model (hereinafter, "prediction model") that indicates a relationship between lifestyle and a check value, using actual data regarding individuals' lifestyle and a check value for each preset check item as training data.

The check value prediction unit 12 acquires actual data regarding the lifestyle of a user, and predicts a future check value of the user by using the acquired actual data and the model. The display unit 13 displays, on a screen, the future check value predicted by the check value prediction unit 12.

Thus, in this embodiment, a future check value in the case if a user were to continue his/her current lifestyle is predicted, and the predicted future check value is provided to the user. The user can then realize the need to improve his/her lifestyle. Accordingly, according to this embodiment, the user can be made aware that his/her health condition will change as a result of improving his/her lifestyle.

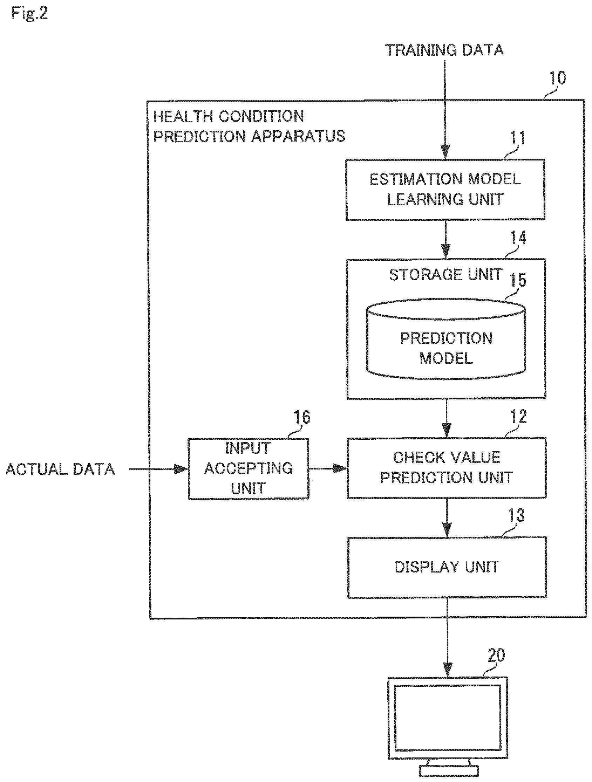

Subsequently, the configuration of the health condition prediction apparatus 10 according to this embodiment will be described in more detail with reference to FIG. 2.

FIG. 2 is a block diagram illustrating a specific configuration of a health condition prediction apparatus according to the embodiment of the present invention.

First, in this embodiment, the health condition prediction apparatus 10 according to this embodiment includes a storage unit 14 and an input accepting unit 16, in addition to the estimation model learning unit 11, the check value prediction unit 12, and the display unit 13, as shown in FIG. 2. The storage unit 14 stores a prediction model 15 that has been obtained through learning performed by the estimation model learning unit 11. The input accepting unit 16 accepts actual data that is input from an external input device, and inputs the accepted actual data to the check value prediction unit 12. The input device may be a keyboard, a touch panel, or the like. However, the input device may also be a terminal device that is connected to the health condition prediction apparatus 10 via a network.

In this embodiment, the estimation model learning unit 11 acquires training data. As mentioned above, the training data is constituted by actual data regarding individuals' lifestyle, and individuals' check values. Also, in this embodiment, it is favorable that the training data is segmented according to when the check values were acquired (one year ago, two years ago, and so on, based on the present time).

Specifically, the actual data regarding lifestyle may be answers to questions about lifestyle. The questions about lifestyle may be the following questions (a) to (h), for example. Answer options are listed in brackets.
(a) How often do you perform exercise that makes you sweat lightly for at least 30 minutes per session? (once or twice a month, once a week, two or three times a week, everyday)
(b) How many meals do you eat a day? (one, two, three, four or more)
(c) Do you eat faster than others? (yes, no)
(d) Do you eat a meal within two hours before going to bed three times or more a week? (yes, no)
(e) Do you have any snacks (late-night snack other than three main meals) after dinner three times or more a week? (yes, no)
(f) Do you skip breakfast three times or more a week? (yes, no)
(g) Do you have a staple, main dish, and side dish at every meal? (yes, no)
(h) Do you eat in moderation? (yes, no)

The check items may be (A) to (I) below, for example.
(A) HbAlc
(B) fasting blood glucose
(C) neutral fat
(D) abdominal girth
(E) HDL cholesterol
(F) LDL cholesterol
(G) weight
(H) systolic blood pressure
(I) diastolic blood pressure

In this embodiment, the estimation model learning unit 11 creates the prediction model 15 that indicates a relationship between lifestyle and each check item based on the acquired training data, using a machine learning algorithm. Also, the estimation model learning unit 11 stores the created prediction model 15 in the storage unit 14.

A machine learning algorithm that can be used in this embodiment may be an existing machine learning algorithm, or may be a machine learning algorithm that is yet to be developed. Specifically, the machine learning algorithm may be, for example, a heterogeneous mixture learning algorithm (see US Patent Application Publication No. 2014/0222741 and JP 2016-509271T).

In the case of using a heterogeneous mixture learning algorithm, the estimation model learning unit 11 initially specifies a pattern of changes in the acquired training data, and divides the original training data into a plurality of pieces of partial data so as to increase the mining accuracy for the specified pattern. The estimation model learning unit 11 then calculates a prediction expression that serves as a prediction model, for each piece of partial data. As a result, patterns and regularities that mixed in the training data are separately extracted, and thus, the prediction accuracy is improved.

In this embodiment, the check value prediction unit 12 acquires the actual data regarding lifestyle of a user via the input accepting unit 16. The actual data acquired from a user may also be answers to the aforementioned questions regarding lifestyle.

The check value prediction unit 12 then applies the acquired actual data to the prediction model 15 stored in the storage unit 14, and predicts future check values of the user, e.g. check values one year, two years, and three years into the future, for example.

In addition, in this embodiment, the check value prediction unit 12 can also determine, using the predicted future check values, the risk of the user suffering from diseases set in advance, e.g. lifestyle-related diseases such as visceral fat obesity, diabetes, hypertension, and hyperlipidemia. Specifically, the check value prediction unit 12 determines the risk of the user suffering from lifestyle-related diseases based on preset rules, as will be described later.

Furthermore, the check value prediction unit 12 can also predict future check values (hereinafter, "post-change check values") in the case if the lifestyle of the user were to change. In this case, the check value prediction unit 12 inputs the post-change check values to the display unit 13.

In this embodiment, the display unit 13 displays the future check values predicted by the check value prediction unit 12, on a screen of a display device 20. If the likelihood of the user suffering from lifestyle-related diseases is calculated, the display unit 13 also displays this likelihood on the screen.

The display device 20 may be a liquid-crystal display device, or the like. Note that, in this embodiment, a terminal device that is connected to the health condition prediction apparatus 10 via a network may be used in place of the display device 20. In this case, the future check values are displayed on a screen of the terminal device.

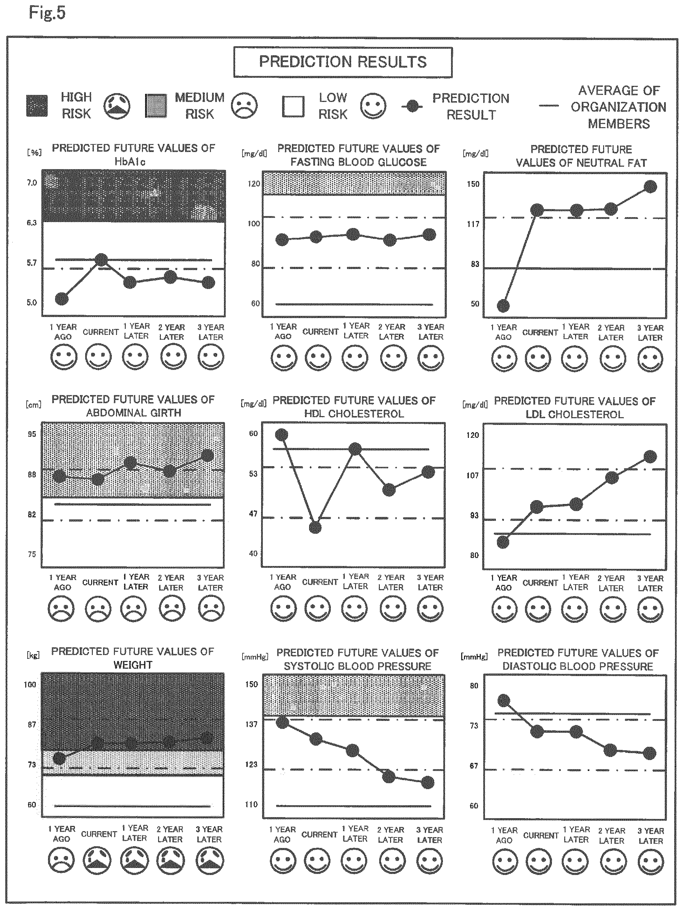

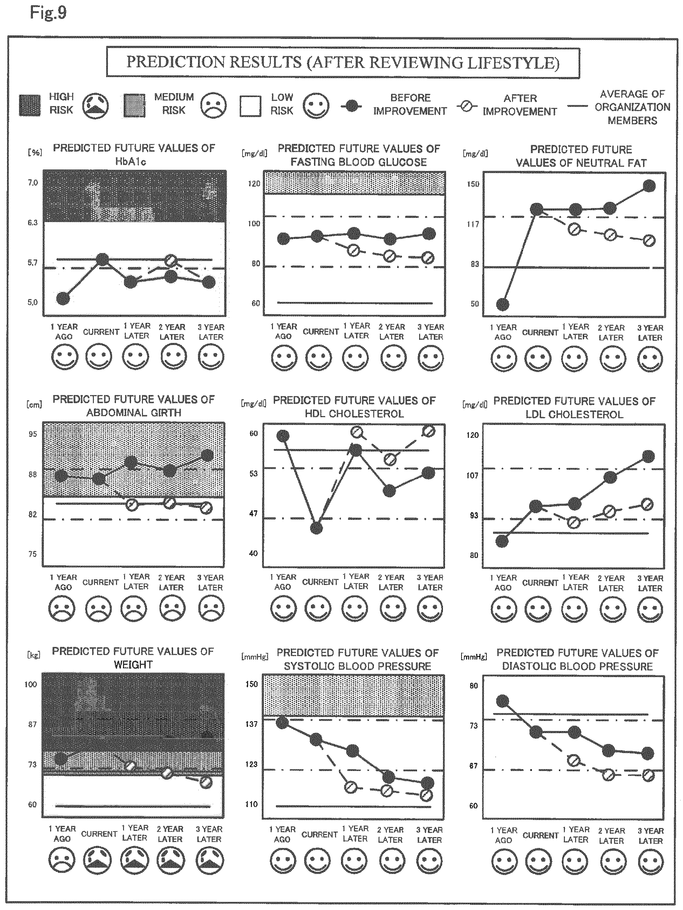

The display unit 13 can also display different icons (see FIG. 5, which will be described later) on the screen in accordance with the post-change check values. Furthermore, if the post-change check values are predicted by the check value prediction unit 12, the display unit 13 displays the initially-predicted check values and the post-change check values. In this case, the display unit 13 can display different icons in accordance with the post-change check values (see FIG. 9, which will be described later).

### Apparatus operation

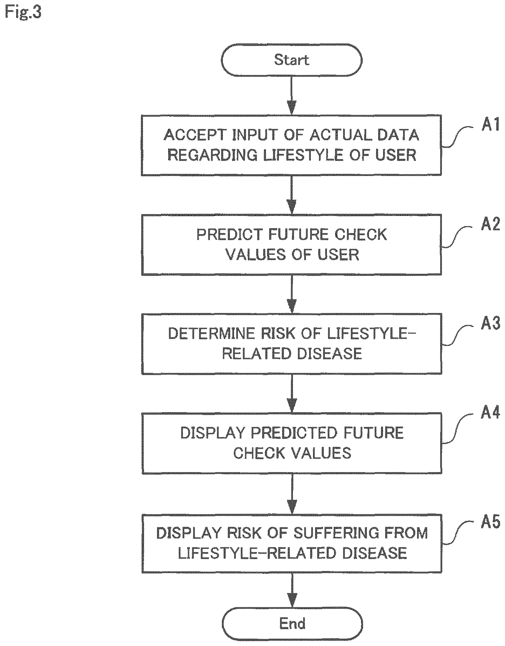

Next, an operation of the health condition prediction apparatus 10 according to the embodiment of the present invention will be described with reference to FIGS. 3 to 9. In this embodiment, a health condition prediction method is carried out by operating the health condition prediction apparatus 10. Accordingly, the following description of the operation of the health condition prediction apparatus 10 will substitute for a description of the health condition prediction method according to this embodiment.

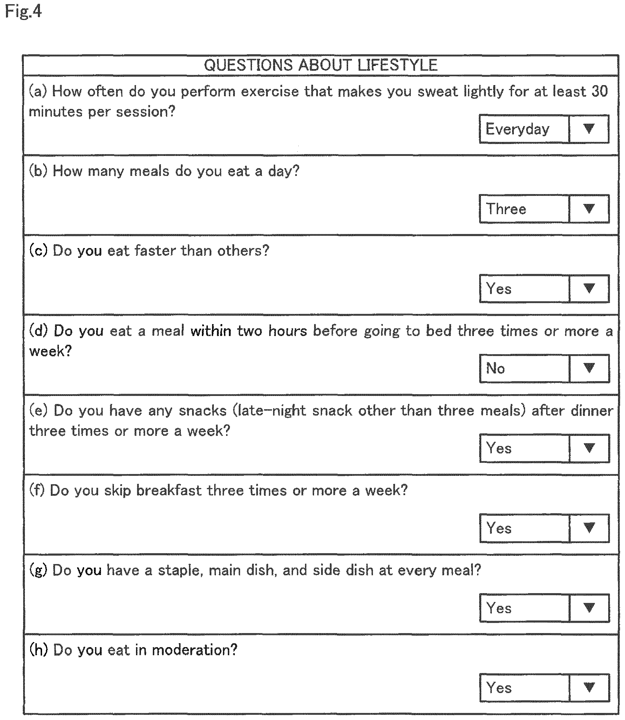

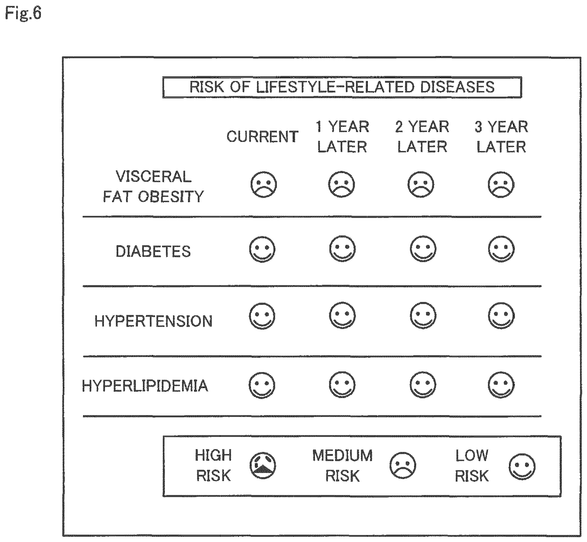

First, processing to predict check values will be described with reference to FIGS. 3 to 6. FIG. 3 is a flowchart illustrating operations during processing to predict check values performed by the health condition prediction apparatus according to the embodiment of the present invention. FIG. 4 shows an example of actual data that is acquired from a user. FIG. 5 shows an example of prediction results. FIG. 6 shows an example of results of determining the risk of suffering from lifestyle-related diseases.

As shown in FIG. 3, initially, the check value prediction unit 12 acquires actual data regarding lifestyle of a user via the input accepting unit 16 (step A1).

Specifically, the check value prediction unit 12 causes the display unit 13 to display the questions regarding lifestyle on the screen, as shown in FIG. 4, and has the user input answers to the questions. The input answers are input to the check value prediction unit 12 via the input accepting unit 16.

Next, the check value prediction unit 12 applies the actual data acquired in step A1 to the prediction model 15 stored in the storage unit 14, and predicts future check values of the user (step A2).

Specifically, the check value prediction unit 12 predicts check values one year, two years, and three years into the future, for example, for check items including HbA1c, fasting blood glucose, neutral fat, abdominal girth, HDL cholesterol, LDL cholesterol, weight, systolic blood pressure, and diastolic blood pressure.

Next, the check value prediction unit 12 determines the risk of the user suffering from lifestyle-related diseases, using the future check values predicted in step A2 (step A3).

Specifically, in this embodiment, risk ranges, namely a high risk range, a medium risk range, and a low risk range are set in the order from high risk of suffering from a lifestyle-related disease, for each check item. Accordingly, the check value prediction unit 12 initially determines the risk range into which each of the predicted check values falls. Note that the risk ranges are set as appropriate by, for example, an administrator of the health condition prediction apparatus 10, based on check values of constituent members of an organization to which the user belongs.

Subsequently, the check value prediction unit 12 determines the risk of the user suffering from lifestyle-related diseases, in accordance with the risk range into which each check value falls. The risk is determined based on preset rules. For example, a rule may be applied in which "the risk range of visceral fat obesity is medium if the risk range of the abdominal girth is medium, is high if the risk range of the abdominal girth is medium or high and the risk range of any of neutral fat, fasting blood glucose, HDL cholesterol, systolic blood pressure, and diastolic blood pressure is medium or high, and is low in other cases".

Next, the display unit 13 receives the future check values predicted in step A2 from the check value prediction unit 12, and displays them on the screen of the display device 20 (step A4).

In this embodiment, the display unit 13 displays, on a screen, the future check values for the respective check items, as well as average values of the constituent members to which the user belongs, and icons (faces), as shown in FIG. 5. Since the risk ranges into which the respective check values fall have been determined by the check value prediction unit 12 in the aforementioned step A3, in the example in FIG. 5, the display unit 13 displays different icons in accordance with the determined risk ranges. That is to say, the design of the icons changes in accordance with the risk range. Furthermore, in the example in FIG. 5, the display unit 13 displays check values from one year ago and the current check values that have been acquired from the user, for the respective check items.

Next, the display unit 13 also displays, on the screen, the risk of the user suffering from lifestyle-related diseases based on the results in step A3, as shown in FIG. 6 (step A5). In the example in FIG. 6, the display unit 13 uses icons to expresses the levels of risk for each disease name and each year, similarly to the example in FIG. 5.

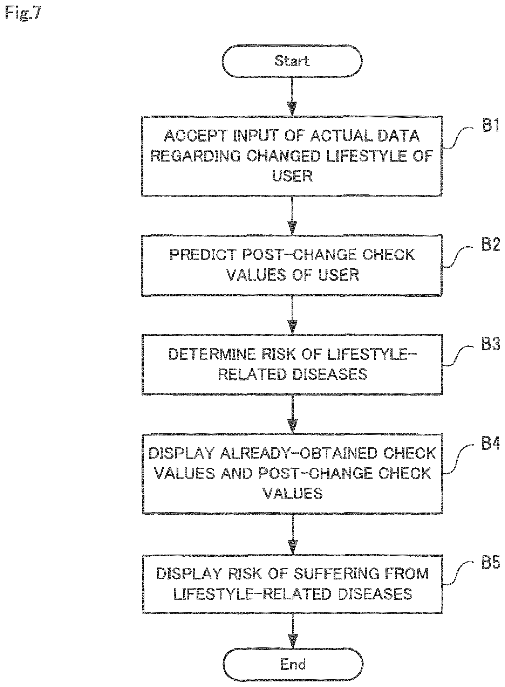

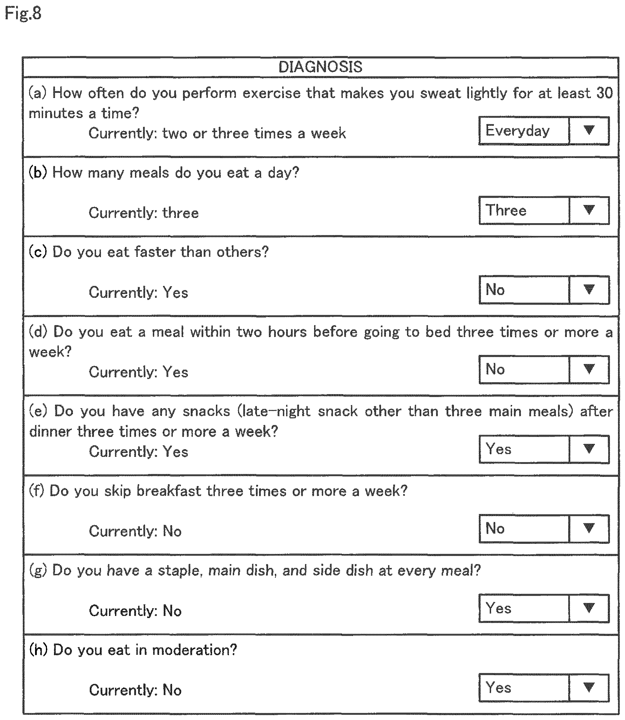

Subsequently, processing to predict post-change check values will be described with reference to FIGS. 7 to 9. FIG. 7 is a flowchart illustrating operations during processing to predict post-change check values performed by the health condition prediction apparatus according to the embodiment of the present invention. FIG. 8 shows an input example of a changed lifestyle. FIG. 9 illustrates an example of results of predicting post-change check values.

As shown in FIG. 7, initially, the check value prediction unit 12 acquires actual data in the case if the user where to change his/her lifestyle, via the input accepting unit 16 (step B1).

Specifically, the check value prediction unit 12 simultaneously causes the display unit 13 to display the questions about lifestyle and the current state on a screen, as shown in FIG. 8, and has the user answer the questions in the case if the user were to change his/her lifestyle. The input answers are input to the check value prediction unit 12 via the input accepting unit 16.

Next, the check value prediction unit 12 applies the post-change actual data acquired in step B1 to the prediction model 15 stored in the storage unit 14, and predicts post-change check values of the user (step B2).

Specifically, the check value prediction unit 12 predicts check values one year, two years, and three years into the future, for example, for the check items such as HbA1c, fasting blood glucose, neutral fat, abdominal girth, HDL cholesterol, LDL cholesterol, weight, systolic blood pressure, and diastolic blood pressure, using the post-change actual data.

Next, the check value prediction unit 12 determines the risk of the user suffering from lifestyle-related diseases, using the post-change check values predicted in step B2 (step B3). Step B3 is performed similarly to step A3 shown in FIG. 3. Accordingly, the check value prediction unit 12 initially determines the risk ranges into which the respective post-change check values fall, and applies the determination results to preset rules to determine the risk of the user suffering from lifestyle-related diseases.

Next, the display unit 13 receives the post-change check values predicted in step B2 from the check value prediction unit 12, and displays the actually-obtained check values and the post-change check values on the screen of the display device 20 (step B4). Also, as shown in FIG. 9, the display unit 13 displays, on the screen, different icons (faces) in accordance with the risk ranges into which the post-change check values predicted in step B2 fall, similarly to the example in FIG. 5. In the example in FIG. 9 as well, the check values from one year ago and the current check values acquired from the user are displayed for each check item.

Next, the display unit 13 also displays the risk of the user suffering from lifestyle-related diseases, based on the results in step B3 (step B5). Step B5 is a step similar to step A5 shown in FIG. 3. In step B5 as well, the display unit 13 expresses the level of risk using an icon design for each disease name and each year, as shown in FIG. 6.

### Effects of the embodiment

As described above, according to this embodiment, the user can understand his/her future health condition at a glance simply by answering questions about lifestyle, and can also realize the need to improve his/her lifestyle. The user can also understand, at a glance, how the check values will change if the user were to change his/her lifestyle, and accordingly, according to this embodiment, the user can be more reliably made to be aware of improvement of his/her lifestyle.

### Modification 1

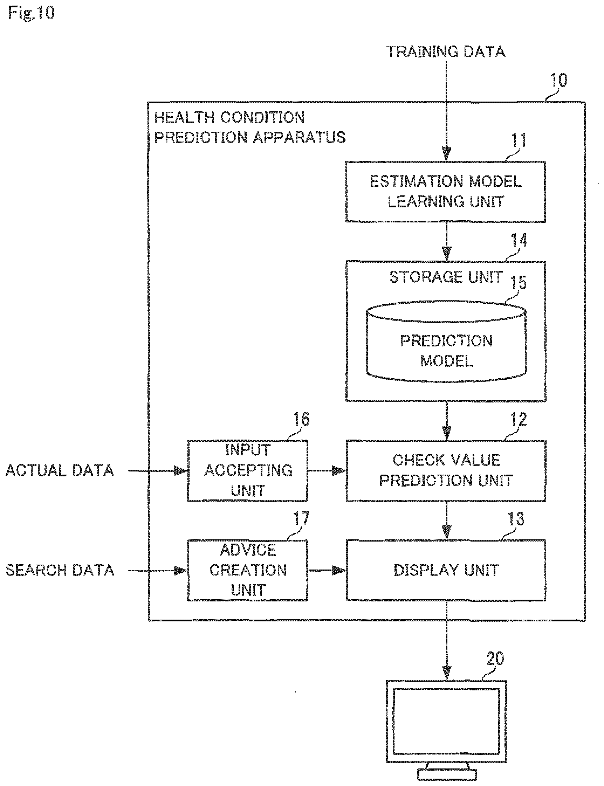

Modification 1 of this embodiment will now be described. FIG. 10 is a block diagram illustrating a configuration of the health condition prediction apparatus according to Modification 1 of the embodiment of the present invention. As shown in FIG. 10, in Modification 1, the health condition prediction apparatus 10 further includes an advice creation unit 17.

The advice creation unit 17 creates advice to be provided to the user, based on user information that has been registered in advance, and presents the created advice to the user. For example, it is assumed that past exercise history and the residential address of the user are registered as user information. In this case, the advice creation unit 17 accesses an external search server to search for sports facilities that are located near the residential address of the user, and specifies a sports facility that coincides with the past exercise history of the user, from among the searched sports facilities. The advice creation unit 17 then causes the display unit 13 to display the specified sports facility on the screen of the display device 20.

For example, if the user belonged to a badminton club when he/she was a student, and there is a gym where he/she can play badminton near the residence of the user, the advice creation unit 17 presents this gym and suggests that the user starts playing badminton. Thus, according to Modification 1, it is possible to assist the user in reconsidering his/her lifestyle.

### Modification 2

In this embodiment, the display unit 13 can display actually-obtained check values and post-change check values for each check item, using graphs that indicate changes in time series, as shown in FIG. 9. At this time, in Modification 2, the display unit 13 can partially change the gap between marks on the vertical axis of the graphs so as to emphasize the difference between the actually-obtained check values and the post-change check values. Specifically, the display unit 13 can expand the gap between marks only in a portion between a post-change check value and an actually-obtained check value so as to emphasize the difference therebetween. According to Modification 2, the user can be clearly made aware of changes brought about by lifestyle, and can further realize the health risk if he/she were to continue his/her current lifestyle.

### Program

A program according to this embodiment may be a program for causing a computer to perform steps A1 to A5 in FIG. 3 and steps B1 to B5 in FIG. 7. By installing this program in the computer and executing it, the health condition prediction apparatus 10 and the health condition prediction method according to this embodiment can be realized. In this case, a CPU (Central Processing Unit) of the computer functions as the estimation model learning unit 11, the check value prediction unit 12, the display unit 13, and the input accepting unit 16, and performs processing.

The program according to this embodiment may also be executed by a computer system that is constituted by a plurality of computers. In this case, for example, each of the computers may function as any of the estimation model learning unit 11, the check value prediction unit 12, the display unit 13, and the input accepting unit 16.

### Physical configuration

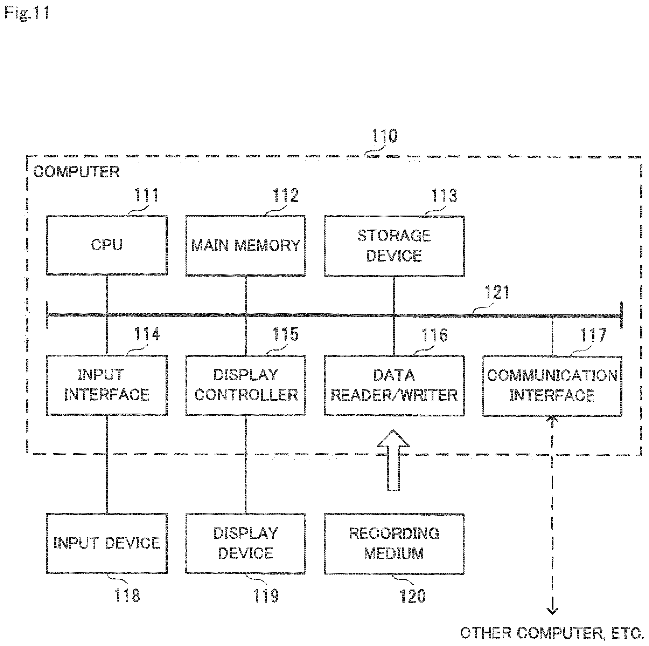

A description will now be given, with reference to FIG. 11, of a computer that realizes the health condition prediction apparatus 10 by executing the program according to this embodiment. FIG. 11 is a block diagram illustrating an example of a computer that realizes the health condition prediction apparatus according to the embodiment of the present invention.

As shown in FIG. 11, a computer 110 includes a CPU 111, a main memory 112, a storage device 113, an input interface 114, a display controller 115, a data reader/writer 116, and a communication interface 117. These units are connected to each other via a bus 121 so as to be able to communicate data.

The CPU 111 loads the program (codes) according to this embodiment that is stored in the storage device 113 to the main memory 112 and executes the codes in a predetermined order, thereby performing various kinds of computation. The main memory 112 typically is a volatile storage device, such as a DRAM (Dynamic Random Access Memory). The program according to this embodiment is provided in a state of being stored in a computer-readable recording medium 120. Note that the program according to this embodiment may also be distributed on the Internet to which the computer is connected via the communication interface 117.

Specific examples of the storage device 113 include a hard disk drive, a semiconductor storage device such as a flash memory, and the like. The input interface 114 mediates data transmission between the CPU 111 and an input device 118 such as a keyboard or a mouse. The display controller 115 is connected to a display device 119 and controls display on the display device 119.

The data reader/writer 116 mediates data transmission between the CPU 111 and the recording medium 120, reads out the program from the recording medium 120, and writes the results of processing performed by the computer 110 in the recording medium 120. The communication interface 117 mediates data transmission between the CPU 111 and other computers.

Specific examples of the recording medium 120 include a general-purpose semiconductor storage device such as a CF (Compact Flash (registered trademark)) or a SD (Secure Digital), a magnetic recording medium such as a Flexible Disk, and an optical storage medium such as a CD-ROM (Compact Disk Read Only Memory).

The health condition prediction apparatus 10 according to this embodiment may also be realized by using hardware that corresponds to each of the units, rather than a computer in which the program is installed. Furthermore, the health condition prediction apparatus 10 may be partially realized by a program, and the remainder may be realized by hardware.

The above-described embodiment can be expressed, partially or entirely, by the following Note 1 to Note 15, but is not limited thereto.

### Supplementary Note 1

A health condition prediction apparatus including:
an estimation model learning unit for learning a model indicating a relationship between lifestyle and a check value for a preset check item, using actual data regarding individuals' lifestyle and the check value as training data;
a check value prediction unit for acquiring actual data regarding lifestyle of a user, and predicting a future check value of the user by using the acquired actual data and the model; and
a display unit for displaying, on a screen, the future check value predicted by the check value prediction unit.

### Supplementary Note 2

The health condition prediction apparatus according to Supplementary Note 1,
wherein the display unit displays different icons in accordance with the future check value predicted by the check value prediction unit.

### Supplementary Note 3

The health condition prediction apparatus according to Supplementary Note 1 or 2,
wherein the check value prediction unit also predicts a future check value in a case if the lifestyle of the user changes, and
the display unit displays, as results of the prediction, the future check value and the future check value in the case if the lifestyle of the user changes, and further displays different icons in accordance with the future check value in the case if the lifestyle of the user changes.

### Supplementary Note 4

The health condition prediction apparatus according to Supplementary Note 3,
wherein the display unit displays the future check value and the future check value in the case if the lifestyle of the user changes, using a graph that indicates a change in time series, and at this time, the display unit partially changes an interval between marks on a vertical axis of the graph so as to emphasize a difference between the future check value and the future check value in the case if the lifestyle of the user changes.

### Supplementary Note 5

The health condition prediction apparatus according to any one of Supplementary Notes 1 to 4, further including:
an advice creation unit for creating advice to be provided to the user, based on user information regarding the user, and presenting the created advice to the user.

### Supplementary Note 6

A health condition prediction method including:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b)

### Supplementary Note 7

The health condition prediction method according to Supplementary Note 6,
wherein, in the step (c), different icons are displayed in accordance with the future check value predicted in the step (b).

### Supplementary Note 8

The health condition prediction method according to Supplementary Note 6 or 7,
wherein, in the step (b), a future check value in a case if the lifestyle of the user changes is also predicted, and
in the step (c), the future check value and the future check value in the case if the lifestyle of the user changes are displayed as results of the prediction, and furthermore, different icons are displayed in accordance with the future check value in the case if the lifestyle of the user changes.

### Supplementary Note 9

The health condition prediction method according to Supplementary Note 8,
wherein, in the step (c), the future check value and the future check value in the case if the lifestyle of the user changes are displayed using a graph that indicates a change in time series, and at this time, an interval between marks on a vertical axis of the graph is partially changed so as to emphasize a difference between the future check value and the future check value in the case if the lifestyle of the user changes.

### Supplementary Note 10

The health condition prediction method according to any one of Supplementary Notes 6 to 9, further including:
a step (d) of creating advice to be provided to the user, based on user information regarding the user, and presenting the created advice to the user.

### Supplementary Note 11

A computer-readable recording medium storing a program including a command for causing a computer to perform:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b)

### Supplementary Note 12

The computer-readable recording medium according to Supplementary Note 11,
wherein, in the step (c), different icons are displayed in accordance with the future check value predicted in the step (b).

### Supplementary Note 13

The computer-readable recording medium according to Supplementary Note 11 or 12,
wherein, in the step (b), a future check value in a case if the lifestyle of the user changes is also predicted, and
in the step (c), the future check value and the future check value in the case if the lifestyle of the user changes are displayed as results of the prediction, and furthermore, different icons are displayed in accordance with the future check value in the case if the lifestyle of the user changes.

### Supplementary Note 14

The computer-readable recording medium according to Supplementary Note 13,
wherein, in the step (c), the future check value and the future check value in the case if the lifestyle of the user changes are displayed using a graph that indicates a change in time series, and at this time, an interval between marks on a vertical axis of the graph is partially changed so as to emphasize a difference between the future check value and the future check value in the case if the lifestyle of the user changes.

### Supplementary Note 15

The computer-readable recording medium according to any one of Supplementary Notes 11 to 14,
wherein the program further includes a command for causing the computer to perform:
a step (d) of creating advice to be provided to the user, based on user information regarding the user, and presenting the created advice to the user.

Although the invention of the present application has been described with reference to the embodiment, the invention of the present application is not limited to the above-described embodiment. Configurations and details of the invention of the present application may be changed in various manners that can be understood by those skilled in the art, within the scope of the invention of the present application.

Although the invention of the present application has been described with reference to the embodiment, the invention of the present application is not limited to the above-described embodiment. Configurations and details of the invention of the present application may be changed in various manners that can be understood by those skilled in the art, within the scope of the invention of the present application.

This application claims priority of Japanese Patent Application No. 2016-102719 filed May 23, 2016, the entire disclosure of which is incorporated herein.

### INDUSTRIAL APPLICABILITY

As described above, the present invention can make a user aware that his/her health condition will change as a result of improving his/her lifestyle. The present invention is useful in health management-related fields.

### REFERENCE SIGNS LIST

- 10: Health condition prediction apparatus
- 11: Estimation model learning unit
- 12: Check value prediction unit
- 13: Display unit
- 14: Storage unit
- 15: Prediction model
- 16: Input accepting unit
- 20: Display device
- 110: Computer
- 111: CPU
- 112: Main memory
- 113: Storage device
- 114: Input interface
- 115: Display controller
- 116: Data reader/writer
- 117: Communication interface
- 118: Input device
- 119: Display device
- 120: Recording medium
- 121: Bus

## Claims

1. A health condition prediction apparatus comprising:
an estimation model learning unit for learning a model indicating a relationship between lifestyle and a check value for a preset check item, using actual data regarding individuals' lifestyle and the check value as training data;
a check value prediction unit for acquiring actual data regarding lifestyle of a user, and predicting a future check value of the user by using the acquired actual data and the model; and
a display unit for displaying, on a screen, the future check value predicted by the check value prediction unit.

2. The health condition prediction apparatus according to claim 1,
wherein the display unit displays different icons in accordance with the future check value predicted by the check value prediction unit.

3. The health condition prediction apparatus according to claim 1 or 2,
wherein the check value prediction unit also predicts a future check value in a case if the lifestyle of the user changes, and
the display unit displays, as results of the prediction, the future check value and the future check value in the case if the lifestyle of the user changes, and further displays different icons in accordance with the future check value in the case if the lifestyle of the user changes.

4. The health condition prediction apparatus according to claim 3,
wherein the display unit displays the future check value and the future check value in the case if the lifestyle of the user changes, using a graph that indicates a change in time series, and at this time, the display unit partially changes an interval between marks on a vertical axis of the graph so as to emphasize a difference between the future check value and the future check value in the case if the lifestyle of the user changes.

5. The health condition prediction apparatus according to any one of claims 1 to 4, further comprising:
an advice creation unit for creating advice to be provided to the user, based on user information regarding the user, and presenting the created advice to the user.

6. A health condition prediction method comprising:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b).

7. The health condition prediction method according to claim 6,
wherein, in the step (c), different icons are displayed in accordance with the future check value predicted in the step (b).

8. The health condition prediction method according to claim 6 or 7,
wherein, in the step (b), a future check value in a case if the lifestyle of the user changes is also predicted, and
in the step (c), the future check value and the future check value in the case if the lifestyle of the user changes are displayed as results of the prediction, and furthermore, different icons are displayed in accordance with the future check value in the case if the lifestyle of the user changes.

9. The health condition prediction method according to claim 8,
wherein, in the step (c), the future check value and the future check value in the case if the lifestyle of the user changes are displayed using a graph that indicates a change in time series, and at this time, an interval between marks on a vertical axis of the graph is partially changed so as to emphasize a difference between the future check value and the future check value in the case if the lifestyle of the user changes.

10. The health condition prediction method according to any one of claims 6 to 9, further comprising:
a step (d) of creating advice to be provided to the user, based on user information regarding the user, and presenting the created advice to the user.

11. A computer-readable recording medium storing a program including a command for causing a computer to perform:
a step (a) of learning a model indicating a relationship between lifestyle and a check value for a preset check item, using actual data regarding individuals' lifestyle and the check value as training data;
a step (b) of acquiring actual data regarding lifestyle of a user and predicting a future check value of the user by using the acquired actual data and the model; and
a step (c) of displaying, on a screen, the future check value predicted in the step (b).

12. The computer-readable recording medium according to claim 11,
wherein, in the step (c), different icons are displayed in accordance with the future check value predicted in the step (b).

13. The computer-readable recording medium according to claim 11 or 12,
wherein, in the step (b), a future check value in a case if the lifestyle of the user changes is also predicted, and
in the step (c), the future check value and the future check value in the case if the lifestyle of the user changes are displayed as results of the prediction, and furthermore, different icons are displayed in accordance with the future check value in the case if the lifestyle of the user changes.

14. The computer-readable recording medium according to claim 13,
wherein, in the step (c), the future check value and the future check value in the case if the lifestyle of the user changes are displayed using a graph that indicates a change in time series, and at this time, an interval between marks on a vertical axis of the graph is partially changed so as to emphasize a difference between the future check value and the future check value in the case if the lifestyle of the user changes.

15. The computer-readable recording medium according to any one of claims 11 to 14,
wherein the program further includes a command for causing the computer to perform:
a step (d) of creating advice to be provided to the user, based on user information regarding the user, and presenting the created advice to the user.
